(19) 

**Europäisches Patentamt**

**European Patent Office**

**Office européen des brevets**

(11) **EP 4 732 891 A1**

(12) **EUROPEAN PATENT APPLICATION**

(43) Date of publication:
**29.04.2026 Bulletin 2026/18**

(21) Application number: **24383161.7**

(22) Date of filing: **22.10.2024**

(51) International Patent Classification (IPC):
**A61N 1/36** *(2006.01)* **A61N 1/32** *(2006.01)*
**A61B 18/12** *(2006.01)*

(52) Cooperative Patent Classification (CPC):
**A61N 1/32; A61B 18/1233; A61N 1/36034;**
**A61N 1/36071;** A61B 2018/0016;
A61B 2018/00613; A61B 2018/0072;
A61B 2018/00726; A61B 2018/00732;
A61B 2018/00767; A61B 2018/00791;
A61B 2018/00815; A61B 2018/00821;
A61B 2018/128; A61B 2018/162; (Cont.)

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**GE KH MA MD TN**

(71) Applicant: **Universitat Pompeu Fabra**
**08002 Barcelona (ES)**

(72) Inventor: **IVORRA CANO, Antoni**
**08002 Barcelona (ES)**

(74) Representative: **TRBL Intellectual Property**
**Plaza de Castilla 3, 7°A**
**28046 Madrid (ES)**

(54) **IMPLANT DEVICE AND SYSTEM FOR PULSED RADIOFREQUENCY TREATMENTS**

(57)     The present invention relates to an implant device (1) suitable for treatments based on delivering pulsed radiofrequency (PRF) electric fields to a treatment target area (2) of a subject's body. The implant device (1) comprises at least a plurality of primary electrodes (4) and an electronic circuit (5) connected to said plurality of primary electrodes (4). The plurality of primary electrodes (4) comprises at least a volume conduction electrode pair (4') comprised by a treatment electrode (4") and a return electrode (4'''), which are adapted to pick-up and channel input electrical energy flowing through a tissue of the subject's body in the form of a plurality of radiofrequency electric current pulses (16). The electronic circuit (5) is adapted to regulate said input electrical energy and deliver pulsed radiofrequency electric fields, through the treatment electrode (4"), to the treatment target area (2) of the subject's body. The invention also relates to a system (9) for applying treatments based on delivering pulsed radiofrequency electric fields, which comprises an implant device (1).

**FIG. 1**

(Cont. next page)

(52) Cooperative Patent Classification (CPC): (Cont.)
   A61N 1/326; A61N 1/327; A61N 1/36021;
   A61N 1/3605; A61N 1/37223

**Description**

**FIELD OF THE INVENTION**

The present invention belongs to the field of medical technologies. More specifically, the invention relates to an implant device suitable for treatments based on delivering pulsed radiofrequency (PRF) electric fields. The invention also relates to a system for applying treatments based on delivering pulsed radiofrequency (PRF) electric fields, comprising an implant device according to any of the embodiments described in this document.

**BACKGROUND OF THE INVENTION**

Neuropathies and musculoskeletal pain disorders are among the most common conditions encountered in clinical practice. Conventionally, various treatments such as pain medications, neuropathic agents, muscle relaxants, steroid injections, and nerve blocks are applied to manage said pain disorders. However, these conventional treatments have several adverse effects and tend to relief pain for a short time, and, in many cases, pain relief is incomplete.

Over the years, several electrical neuromodulation methods have been developed for pain management. An important set of these methods is based on delivering current or voltage pulses at relatively low frequencies (<20 kHz, and most typically < 200 Hz) for triggering action potentials in nerves (i.e., for performing electrical stimulation). Another set of techniques is based on delivering radiofrequency (> 50 kHz) currents or voltages to generate an electric field that does not cause electrical stimulation. Depending on how the radiofrequency currents or voltages are administrated, these pain management therapies based on radiofrequency field delivery can be classified into continuous radiofrequency (CRF) or pulsed radiofrequency (PRF). Typically, both CRF and PRF can be performed with the same radiofrequency system and involve the insertion of a needle connected to a radiofrequency generator. The needle is placed next to the region to be treated, in such a way that the tip of the needle transmits the electrical energy generated by the radiofrequency generator to the region to be treated. Thus, by placing the tip of the needle next to the region to be treated it is possible to reduce the pain sensation. Generally, the needle electrode is inserted under local anaesthesia and guided by ultrasound guidance, fluoroscopic guidance or anatomical landmarks.

CRF exposes the region to be treated to high temperatures (70 °C - 90°C) via Joule heating. With this technique, the region to be treated is ablated. The thermal ablation of the region to be treated is thought to be the main mechanism of pain reduction after CRF. The tip of the probe is at approximately 80 °C and induces coagulative necrosis into the region to be treated around the tip. In contrast, it is known that PRF does not expose the region to be treated to high temperatures.

PRF uses a radiofrequency current or voltage applied in the form of bursts (i.e., pulses) with a short duration (e.g., 20 ms) and a relatively long resting phase (e.g., 480 ms). PRF modulates pain signals without causing tissue damage. The mechanism of action is not completely understood but evidence suggests that the effectiveness of PRF is more dependent on the magnitude of the generated electric field in the tissue rather than on the dissipated power. It has been hypothesized that the PRF mechanism of action is related to extracellular calcium uptake and that such uptake is due to electroporation.

The neuromodulatory potential of PRF extends beyond pain management and can, in principle, be applied to treating various conditions that benefit from neuromodulation. These include motor disorders like tremor, dystonia, or spasticity; cognitive disorders such as Parkinson's disease; and other conditions such as obesity, epilepsy, depression, urinary or fecal incontinence, or sexual dysfunction.

PRF has a superior safety profile compared to CRF. However, an important drawback of PRF treatments is that their effect is in most cases temporary, ranging from weeks to several months.

Pulsed radiofrequency fields can also be applied to perform treatments not based on neuromodulation such as wound healing and bone fracture healing. In particular, pulsed radiofrequency fields can be applied to perform treatments based on electroporation. Electroporation is the phenomenon by which cell membrane permeability to ions and molecules is increased when the cell is briefly exposed to high electric fields. Electroporation is the basis of several therapeutic approaches such as gene electrotransfer for, for instance, DNA vaccination, electrochemotherapy and irreversible electroporation for treating tumors, and pulsed field ablation (PFA) for managing cardiac arrhythmias. While these electroporation-based treatments are typically applied in a single session, there are instances where they need to be repeated. Furthermore, repeated mild electroporation can be applied continuously to living tissues to alter their behavior. This alteration may be triggered by the uptake of extracellular calcium, which acts as a secondary messenger within the cell. By setting the frequency and intensity of calcium uptake triggered by adjusting the pulsed radio frequency fields, various cellular processes can be controlled. For instance, cell proliferation can be halted to inhibit tumor growth, or conversely, promoted to accelerate injury repair. Similarly, this approach can also promote cell differentiation, which can be applied for tissue regeneration and repair. Furthermore, it may also regulate cell metabolism and stimulate the release of neurotransmitters and hormones, potentially influencing metabolic processes, immune responses, or other physiological functions, opening possibilities for targeted therapeutic interventions.

In view of the above, there is a need in the field of medical technologies to develop a system that allows repeating the delivery of pulsed radiofrequency electric fields to inner body regions in a controlled and minimally invasive way. This can be achieved by electronic implants. However, because of energy storage and transmission limitations, current implants are unable to generate pulsed radiofrequency fields of sufficient magnitude over extended periods of time.

**BRIEF DESCRIPTION OF THE INVENTION**

A first object of the present invention relates to an implant device, adapted to be implanted inside a subject's body for delivering pulsed radiofrequency electric fields to a treatment target area of said subject's body. This implant device comprises at least a plurality of electrodes and an electronic circuit. More specifically, the implant device preferably comprises:

- a plurality of primary electrodes, comprising at least a volume conduction electrode pair adapted to pick-up and channel an input electrical energy flowing through a tissue of the subject's by volume conduction in the form of a plurality of radiofrequency electric current pulses, wherein the at least volume conduction electrode pair comprises a treatment electrode and a return electrode; and
- an electronic circuit electrically connected to the plurality of primary electrodes and adapted to conduct and regulate the input electrical energy, and deliver an output electrical energy in the form of a plurality of radiofrequency pulses.

The electrical energy flowing through the tissue of the subject's body in the form of a plurality of radiofrequency electric current pulses comprises a fundamental frequency substantially between 50 kHz and 500 MHz and an electric field amplitude substantially between 1 V/cm and 1000 V/cm at the location of the implant device. Advantageously in the invention, the plurality of primary electrodes and the electronic circuit are further adapted to deliver pulsed radiofrequency electric fields to the treatment target area of the subject's body through the treatment electrode, and wherein a resulting voltage waveform between the treatment electrode and the return electrode comprises an amplitude substantially between 5 V and 500 V, and preferably a fundamental frequency substantially between 50 kHz and 1 MHz (without limitation to other fundamental frequency ranges). In a further embodiment of the invention, the resulting voltage waveform comprises an amplitude substantially between 20 V and 400 V. In a further embodiment of the invention, the resulting voltage waveform comprises an amplitude substantially between 20 V and 300 V. In a further embodiment of the invention, the resulting voltage waveform comprises an amplitude substantially between 20 V and 200 V. In a further embodiment of the invention, the resulting voltage waveform comprises an amplitude substantially between 20 V and 100 V. In the present invention, the treatment electrode preferably refers to the electrode positioned closer to the treatment target area of the subject's body than other electrodes in the implant, during the operation thereof. In the present invention, the expression "resulting voltage" refers to a resultant voltage range from the product of the applied current times the bioelectrical impedance of the tissue.

In a preferred embodiment of the invention, the electronic circuit further comprises the following elements:

- a digital control unit adapted to control the delivery of the pulsed radiofrequency electric fields to the treatment target area; and
- a power stage adapted to extract a power by collecting at least part of the input electrical energy and to supply this power to the elements comprised within the electronic circuit.

In a further embodiment of the invention, the electronic circuit further comprises communication means adapted to receive or send information.

In a further preferred embodiment of the invention, the implant device comprises monitoring means, adapted to monitor one or more parameters related to the delivery of pulsed radiofrequency electric fields to the treatment target area and/or to the tissue of the subject's body. In a further preferred embodiment, the electronic circuit is adapted to modulate the delivery of pulsed radiofrequency electric fields to the treatment target area in response to these monitored parameters.

In a further preferred embodiment of the invention, the monitoring means comprises a temperature sensor. The temperature sensor allows the temperature to be measured to ensure no thermal damage is caused on subject's body during the delivery of pulsed radiofrequency electric fields. The temperature sensor may be embedded in the implant device, preferably close to the treatment electrode, in order to control the delivery of pulsed radiofrequency electric fields.

In a further preferred embodiment of the invention, the temperature sensor comprises a thermistor, a thermocouple, and/or a resistance temperature detector.

In a further preferred embodiment of the invention, the implant device comprises at least a plurality of secondary electrodes in order to deliver the pulsed radiofrequency electric fields and direct these fields to the treatment target area of the subject's body.

In a further embodiment of the invention, the electronic circuit is embedded in at least one of the plurality of primary electrodes.

In a further embodiment of the invention, the implant device comprises a substantially elongated flat shape or a substantially elongated tubular shape. Advantageously, with a substantially elongated shape it is possible to deploy the implant device by means of tubular instruments in a minimally invasive manner. In addition, with a substantially elongated flat shape it is possible to deploy the implant device precisely in a treatment target area close to a fascia or between two tissues by sliding the implant device after a small surgical incision and easily secured with surgical means (such as surgical tacks or sutures among others). With a substantially elongated tubular shape it is possible to deploy the implant device by means of instruments such as needles (i.e., by injection techniques).

In a further embodiment of the invention, the treatment target area of the implant device comprises a target nerve.

In a further embodiment of the invention, the electronic circuit is enclosed in an electronic package. More preferably, the electronic package comprises a hermetic capsule.

In a further embodiment of the invention, the implant device is disposed or arranged on a dielectric substrate. In a further embodiment of the invention, the electronic circuit and the plurality of primary electrodes are embedded into a flexible material. More preferably, the flexible material comprises a flexible biomaterial or a flexible thermoplastic, adapted for the fabrication of the implant device by means of photolithography. Even more preferably, the flexible biomaterial comprises silicone and the flexible thermoplastic comprises polyethylene terephthalate.

In a further embodiment of the invention, the electronic circuit comprises a current limiting circuit adapted to limit the flow of the plurality of radiofrequency electric current pulses. Preferably, the current limiting circuit comprises a positive temperature coefficient (PCT) resistor, two antiparallel current limiting diodes (CLD), two antiparallel CLDs with PCT resistors, or a digitally controllable current-limiting circuit.

In a further embodiment of the invention, the electronic circuit comprises a conversion circuit. Preferably, the conversion circuit comprises a rectifier or a pulse generator. More preferably, the rectifier comprises a diode connected in series with a capacitor and in parallel with a resistor, a diode that toggles its polarity after each burst, or a digitally controllable smart rectifier.

A second object of the present invention relates to a system for delivering pulsed radiofrequency fields to a treatment target area of a subject's body, which comprises at least an implant device according to any one of the embodiments described in this document and an external unit.

In a preferred embodiment of the invention, the external unit comprises a plurality of external electrodes adapted to encompass a region of a subject's body comprising a treatment target area, and an external electronic circuit comprising at least a generator electrically connected to the plurality of external electrodes.

Advantageously in the invention, the generator of the system is adapted to generate a plurality of radiofrequency electric pulses comprising:

- a fundamental frequency substantially between 50 kHz and 500 MHz; and/or
- a resulting voltage amplitude between 20 V and 1000 V.

The generator is adapted to supply radiofrequency electric pulses (i.e., AC electric field pulses or busts). As stated, the AC electric field is pulsed (i.e., applied in the form of bursts) rather than continuous, which allows for the delivery of relatively large magnitude fields through the subject's body in a safer manner.

The fundamental frequency of radiofrequency electric pulses generated by the generator is, preferably, larger than 50 kHz and lower than 500 MHz.

The resulting voltage amplitude of the radiofrequency electric pulses generated by the generator is larger than substantially 20 V, yet lower than substantially 1000 V.

The AC electric fields (with fundamental frequency f and with amplitude A) generated by the generator of the invention are advantageously pulsed, i.e., they consist of burst delivered with duration B, and repetition frequency F. The product $F \times B$ is the duty cycle (D).

In a further preferred embodiment of the invention, the generator is programmed to supply the radiofrequency electric pulses during two or more active phases, each of said active phases being separated from the previous and next active phase by a period of time corresponding to a rest phase in which no radiofrequency electric pulses are supplied.

In a further preferred embodiment of the invention, the radiofrequency electric pulses comprise a sinusoidal waveform or a waveform without a DC component (such as symmetric or asymmetric biphasic square pulses, saw pulses, triangular pulses, etc.,) to prevent electrochemical reactions at the electrodes.

In a further preferred embodiment of the invention, the system further comprises amplitude modulation means such that the amplitude of the radiofrequency electric pulses can be modulated. This allows the pulses to comprise a soft start and/or soft end such that harmonics and thus electromagnetic compatibility issues and unsought electrostimulation are minimized.

In a further embodiment of the invention, the external electronic circuit comprises at least the following elements:

- an external digital control unit adapted to automatically control the delivery of the plurality of radiofrequency electric pulses generated by the generator; and
- an external power stage adapted to power the elements comprised within the external electronic circuit.

In a further embodiment of the invention, the external electronic circuit comprises communication means adapted to receive information generated by the implant device or adapted to send information to the implant device.

In a further embodiment of the invention, the external electronic circuit comprises a monitoring unit. Preferably, the monitoring unit comprises a sensor and, more preferably, the sensor comprises an electric sensor.

In a further embodiment of the invention, the plurality of external electrodes of the generator is arranged in pairs such that the radiofrequency electric pulses deployed through said arrangements can be switched in order to align the electric fields.

A third object of the present invention relates to a method for controlling the delivery of pulsed radiofrequency fields delivered by an implant device in a system according to particular embodiments disclosed in this document, wherein the method comprises the realization of the following steps:

- establishing a maximum temperature ($T_{max}$) that can be reached at a treatment target area, and a reference safe temperature ($T_{safe}$);
- measuring a temperature (T) at a treatment target area, by means of a temperature sensor comprised in the implant device;
- transferring information, related to the T measured, to a digital control unit comprised in the implant device, or to an external digital control unit;
- comparing T to $T_{max}$, by the digital control unit;

1) If $T \geq T_{max}$, the digital control unit stops the delivery of the pulsed radiofrequency field and measures again T, and then compares said T to $T_{safe}$;

a) If $T \leq T_{safe}$, a second parameter (SP) is calculated, then being compared to a maximum second parameter threshold ($SP_{max}$);

i. If $SP \geq SP_{max}$, the digital control unit stops the delivery of the pulsed radiofrequency filed;
ii. If $SP \leq SP_{max}$, the delivery of the pulsed radiofrequency field continues, and the control process starts again by measuring T at the treatment target area, by the temperature sensor of the implant device comprised in the system;

b) If $T \geq T_{safe}$, the digital control unit stops the PRF treatment and then step 1) is repeated.

2) If $T \leq T_{max}$, a second parameter (SP) is calculated, then being compared to a maximum second parameter threshold (SP):

i. If $SP \geq SP_{max}$, the digital control unit stops the delivery of the pulsed radiofrequency field; and
ii. If $SP \leq SP_{max}$, the delivery of the pulsed radiofrequency field continues, and the control process starts again by measuring T at the treatment target area, by the temperature sensor of the implant device comprised in the system.

A fourth object of the present invention relates to a method for treating a treatment target area of a subject's body based on the delivery of radiofrequency electric current pulses to a treatment target area of the subject's body, which preferably comprises the operation of a system according to any of the embodiments described in this document.

In a preferred embodiment of the invention, the method comprises the realization of the following steps:

- deploying the implant device in a treatment target area of a subject's body;
- deploying the plurality of external electrodes in a region of the subject's body;
- connecting the plurality of external electrodes to the generator; and
- continuously supplying radiofrequency electric current pulses from the generator to the plurality of external electrodes, wherein said radiofrequency electric current pulses comprise:

- a fundamental frequency between 50 kHz and 500 MHz; and
- an electric field amplitude between 1 V/cm and 1000 V/cm.

In a further preferred embodiment of the invention, the method further comprises a stimulation step, which is applied prior to the radiofrequency electric pulses supply step, in order to confirm the proper deployment of the implant device and the plurality external electrodes connected to the generator. With it is possible to ensure that the implant device is correctly aligned with the target area, thereby maximizing the effectiveness of the treatment. Preferably, the stimulation step comprises the delivery of frequency currents by the implant device.

In a further preferred embodiment of the invention, the treatment target area comprises one or more target nerves.

In the context of the present invention, the term "volume conduction" refers to a method of wireless power transfer in which a subject's own body tissues are used as a channel for the transmission of electrical energy. Thus, in the present invention, the term "volume conduction electrode pair" refers to an electrode pair adapted to pick-up electrical currents which are flowing through a tissue of a subject's body by volume conduction. In the context of the present invention the terms "radiofrequency pulse" and "radiofrequency burst" are used equivalently. Also, in the context of the present invention, "the plurality of external electrodes are adapted to encompass a region of a subject's body" refers primarily to the arrangement of the plurality of the external electrodes in which said region is encompassed by the electric fields generated between the plurality of the external electrodes. In the context of the present invention, the expression "conduct a PRF input" refers to acquire the plurality of radiofrequency electric current pulses, picked-up by the at least volume conduction electrode pair, by the electronic circuit. In the context of the present invention, the term "regulate said PRF input" refers to adjust said PRF input. In the context of the present invention, the term "substantially", applied to any of the terms used herein, shall be understood as identical or within a margin of variation of 5% higher or lower.

All the terms and embodiments described anywhere in this document are equally applicable to all aspects of the invention. The described embodiments shall be understood as intended for combination with other embodiments, unless express exclusion or technical impossibility. It should be noted that, as used in the specification and in the appended claims, the singular forms "a", "an", and "the" include their plural referents unless the context clearly indicates otherwise. Similarly, the term "comprises" or "comprising" as used herein also describes "consists of" or "consisting of" in accordance with generally accepted patent practice.

## DESCRIPTION OF THE DRAWINGS

The foregoing and other features and advantages will be more fully understood from the detailed description of the invention, as well as from examples referring to the attached figures, which are described in the following paragraphs, wherein:

Figure 1 shows a schematic representation of the implant device according to an embodiment of the invention in the context of a preferred application of said implant device for delivering pulsed radiofrequency electric fields, in which the implant device comprises a substantially elongated tubular shape and a temperature sensor is embedded in the treatment electrode.

Figure 2 shows a schematic representation of the implant device according to an embodiment of the invention which comprises a substantially elongated tubular shape and a plurality of secondary electrodes.

Figure 3 shows a schematic representation of the implant device according to an embodiment of the invention in which the implant device comprises a substantially elongated tubular shape and the electronic circuit is embedded in the return electrode.

Figure 4 shows a schematic representation of the top view of the implant device according to an embodiment of the invention in which the implant device comprises a substantially elongated flat shape.

Figure 5 shows a schematic representation of a lateral view of the implant device according to an embodiment of the invention, in which the implant device comprises a substantially elongated flat shape and is positioned on the fascia, near to a target nerve.

Figure 6 shows a schematic representation of the system according to an embodiment of the invention in the context of a preferred application of said system for delivering pulsed radiofrequency fields to a treatment target area of a subject's body.

Figure 7 illustrates a pulsed radiofrequency waveform along with some of the basic parameters describing the same: duration of the pulse (B), repetition frequency (F), fundamental frequency (f), and amplitude (A).

Figure 8 illustrates a pulsed radiofrequency waveform in which the amplitude within a pulse is modulated so that a soft start and a soft end are delivered.

Figure 9 illustrates a system according to an embodiment of the invention in which the external electrodes are arranged in pairs which are selected by switching elements.

Figure 10 shows a schematic representation of an architecture for the external electronic circuit of the external unit comprised in a system according to an embodiment of the invention.

Figure 11 shows a control process flow diagram.

Figure 12 illustrates a schematic representation of the electronic circuit encapsulation of the implant device according to an embodiment of the invention.

Figure 13 shows an implant device according to an embodiment of the invention in which the electronic circuit is hermetically packaged.

Figure 14a-d show schematic representations of different architectures to implement a current limiting circuit (CLC) for direct regulation modality. Figure 14e shows a schematic representation of an architecture for the electronic circuit comprised in an implant device according to an embodiment of the invention for direct regulation mode.

Figure 15a-b shows schematic representations of two different architectures for the electronic circuit comprised in an implant device according to embodiments of the invention for conversion rectifier mode.

Figure 16 shows the operation of the conversion rectifier mode according to an embodiment of the invention.

Figure 17 shows a schematic representation of an architecture for the electronic circuit comprised in an implant device according to an embodiment of the invention for conversion rectifier mode comprising a digitally controllable smart rectifier.

Figure 18 shows the operation of the conversion pulsing mode according to an embodiment of the invention.

Figure 19 shows a schematic representation of an architecture for the electronic circuit comprised in an implant device according to an embodiment of the invention for conversion pulsing mode.

Figure 20 shows a schematic representation of an architecture for the electronic circuit comprised in an implant device according to an embodiment of the invention for conversion pulsing mode.

Numerical references used in the drawings

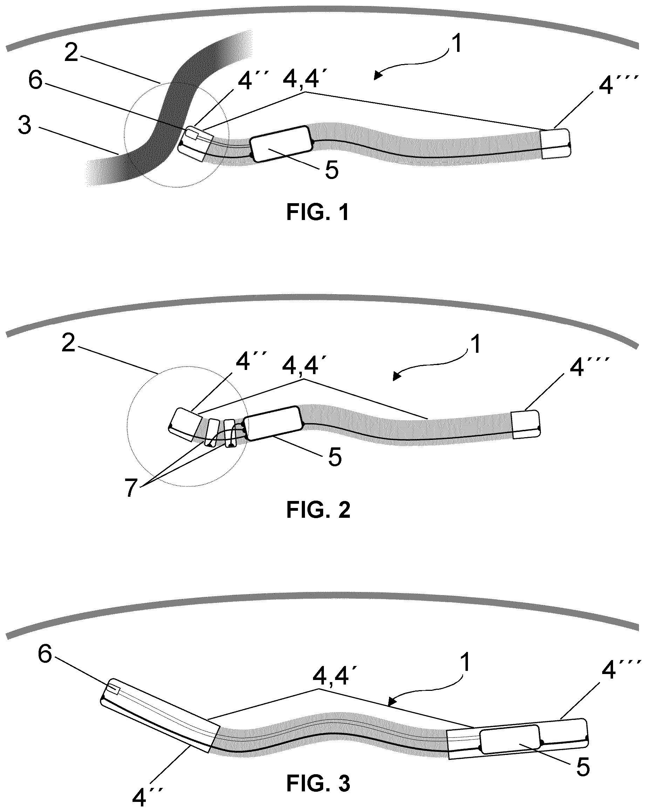

In order to provide a better understanding of the technical features of the invention, the referred Figures 1-20 are accompanied of a series of numerical references which, with an illustrative and non-limiting character, are hereby represented:

| 1 | Implant device |
|---|---|
| 2 | Treatment target area |
| 3 | Target tissue (e.g. a nerve) |
| 4 | Plurality of primary electrodes |
| 4' | Volume conduction electrode pair |
| 4" | Treatment electrode |
| 4'" | Return electrode |
| 5 | Electronic circuit |
| 6 | Temperature sensor |
| 7 | Plurality of secondary electrodes |

(continued)

| | |
|---|---|
| 8 | Dielectric substrate |
| 9 | System |
| 10 | External unit |
| 11 | Plurality of external electrodes |
| 12 | External electronic circuit |
| 13 | Region of subject's body |
| 14 | Generator |
| 15 | Electric field lines |
| 16 | Radiofrequency electric current pulses (or bursts) |
| 17 | Switching elements |
| 18 | External digital control unit (DCU) |
| 19 | Waveform generator |
| 20 | Voltage amplifier |
| 21 | External communication means |
| 22 | Communications means |
| 23 | External power stage |
| 24 | Digital control unit (DCU) |
| 25 | Demodulator |
| 26 | Power stage |
| 27 | Monitoring unit |
| 28 | Electronic sensor |
| 29 | Electronic package |
| 30 | Hermetic capsule |
| 31 | Circuit terminals |
| 31' | Circuit terminals to connect to the plurality of external electrodes |
| 31" | Circuit terminals to connect to the plurality of primary electrodes |
| 31'" | Circuit terminals to connect to the plurality of secondary electrodes |
| 32 | Hermetic electrical connections |
| 33 | Insulation of the connections |
| 34 | Logic gates |
| 34' | Analog gates |
| 35 | Current limiting circuit |
| 35' | Signal conditioning circuit |
| 36 | Pulse generator |
| 36' | Burst detector |
| 37 | Low-pass filtered (LPF) |
| 38 | Intermediate trace |
| 39 | Full bridge rectifier |
| 40 | Voltage regulator |

**DETAILED DESCRIPTION OF THE INVENTION**

The present invention refers to the accompanying drawings, which illustrate specific embodiments in which the present invention may be implemented. These embodiments will be described in detail sufficient to enable those skilled in the art to implement the present invention. It should be understood that various embodiments of the present invention are different from each other but need not be mutually exclusive. Accordingly, the detailed description to be described below is not intended to be taken in a limiting meaning, and the scope of the present invention, if properly described, is limited only by the appended claims, in addition to all scopes equivalent to those claimed by the appended claims. In the drawings, reference numerals refer to the same or similar functions over several aspects.

Figure 1 shows the main elements of an implant device (1) according to an embodiment of the invention, adapted to be implanted inside a subject's body for delivering pulsed radiofrequency fields to a treatment target area (2) thereof, along with a representation of said implant device (1) for its application to the treatment of a condition. As illustrated in this embodiment, the treatment target area (2) comprises a target tissue (3), for instance, a nerve, which causes the disorder to be treated in the subject's body. In another preferred embodiment of the invention, the implant device (1) is applicable for treating conditions that benefit from neuromodulation. These conditions include, but are not limited to, pain, motor disorders such as tremor, dystonia, or spasticity; cognitive disorders such as Parkinson's disease; and other conditions such as obesity, epilepsy, depression, urinary or fecal incontinence, or sexual dysfunction. In another preferred embodiment of the invention, the implant device (1) is applicable for treating conditions not based on neuromodulation such as wound healing and bone fracture healing. In another preferred embodiment of the invention, the implant device (1) is applicable for performing treatments based on electroporation. In another preferred embodiment of the invention, the implant device (1) is applicable for performing treatments based on triggering the uptake of extracellular calcium, which, for instance, can be performed to halt cell proliferation to inhibit tumor growth, or conversely, to promote cell proliferation to accelerate injury repair, or to regulate cell metabolism and stimulate the release of neurotransmitters and hormones, for influencing metabolic processes, immune responses, or other physiological functions.

The implant device (1) comprises a plurality of primary electrodes (4) connected to an electronic circuit (5). The plurality of primary electrodes (4) may consist of at least a volume conduction electrode pair (4'), adapted to pick-up and channel input electrical energy flowing through a tissue of a subject's body by volume conduction in the form of a plurality of radiofrequency electric current pulses (16), comprised in a treatment target area (2) which comprises the target tissue (3). The at least one volume conduction electrode pair (4') comprises a treatment electrode (4") and a return electrode (4‴), both of which pick-up and channel said input electrical energy. Specifically, the treatment electrode (4") is the electrode positioned closer to the treatment target area (2), specifically to the target tissue (3) of the subject's body. In a preferred embodiment of the invention, the volume conduction electrode pair (4') (i.e., the treatment electrode (4") and the return electrode (4‴) may be located at opposite ends of the implant device (1) to maximize the acquisition of input electrical energy. In a preferred embodiment of the invention, the separation distance between the centers of the volume conduction electrode pair (4') (i.e., between the centers of the treatment electrode (4") and the return electrode (4‴) is between 1 cm and 50 cm.

The electronic circuit (5), electrically connected to the plurality of primary electrodes (4) is adapted to conduct an input electrical energy, wherein said input electrical energy flows through a tissue of subject's body where the implant device (1) is located by volume conduction in the form of a plurality of radiofrequency electric current pulses (16) picked-up and channel by the at least volume conduction electrode pair (4') and to regulate said input electrical energy to deliver an output electrical energy (e.g., voltage or current pulsed radiofrequency waveforms) to the treatment target area (2) of the subject's body through the treatment electrode (4").

Figure 1 also shows a temperature sensor (6) connected to the electronic circuit (5), such as a thermistor, a thermocouple, a resistance temperature detector or other known in the art, preferably embedded in the implant device (1) and, more preferably, embedded in the treatment electrode (4"), and adapted to measure the temperature of the treatment target area (2) to ensure no thermal damage is caused on the subject's body during treatment.

Figure 2 shows an embodiment of the invention in which the implant device (1) comprises a plurality of primary electrodes (4) which comprises a volume conduction electrode pair (4') (i.e., a treatment electrode (4") and a return electrode (4‴) and a plurality of secondary electrodes (7). Both the plurality of primary electrodes (4) and the plurality of secondary electrodes (7) are electrically connected to the electronic circuit (5). In this embodiment the volume conduction electrode pair (4') is adapted to pick-up and channel input electrical energy flowing through a tissue of a subject's body by volume conduction in the form of a plurality of radiofrequency electric current pulses (16). On the other hand, the plurality of secondary electrodes (7) is adapted to deliver pulsed radiofrequency fields to the treatment target area (2), specifically to the target tissue (3) and precisely direct the delivery to the target area (2), ensuring that it targets specific areas (2) with high accuracy. By controlling the distribution of the voltage or current waveforms at the secondary electrodes (7), the implant device (1) can achieve therapeutic outcomes with greater precision and reduced side effects, ultimately enhancing the overall efficacy and safety of the treatment.

Figure 3 shows an embodiment of the implant device (1) of the invention which comprises a volume conduction electrode pair (4') (specifically a treatment electrode (4") and a return electrode (4''')) and an electronic circuit (5). Said electronic circuit (5) is embedded in one of the plurality of primary electrodes (4), preferably within the return electrode (4'''). In addition, the implant device (1) also comprises the temperature sensor (6) positioned closer to the treatment target area (2), specifically to the target tissue (3) of the subject's body. In a preferred embodiment of the invention, the electronic circuit (5) is enclosed into an electronic package (29). If the electronic package (29) is made of a conductive material, such as titanium, the electronic package (29) may be used as an electrode.

The implant device (1) as illustrated in Figure 1, Figure 2 and Figure 3 comprises a substantially elongated tubular shape. In another preferred embodiment of the invention, the implant device (1) may comprise a substantially elongated flat shape.

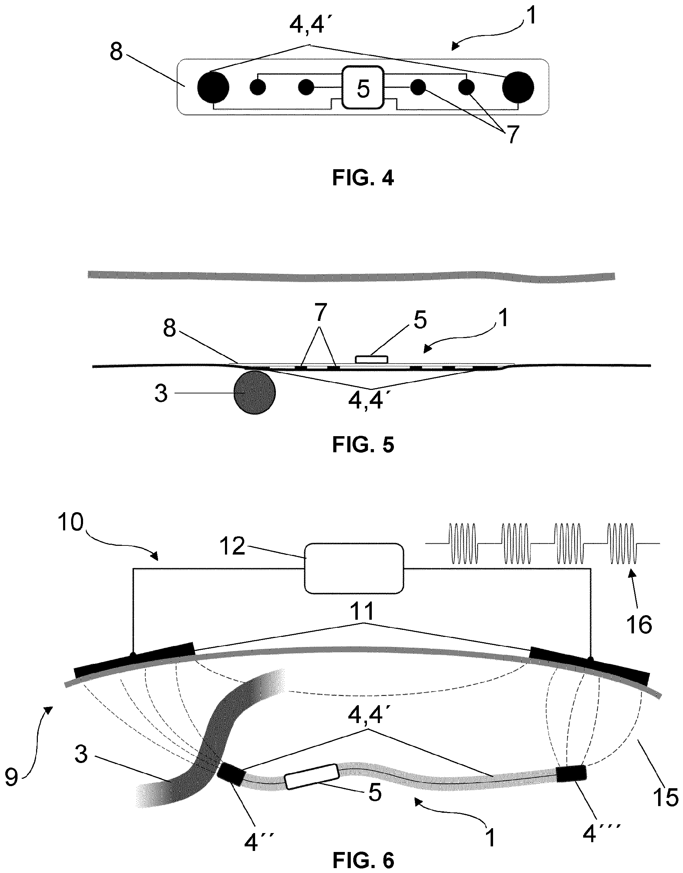

Figure 4 shows a schematic representation of the top view of the implant device (1) according to an embodiment of the invention, in which a plurality of primary electrodes (4), specifically, a volume conduction electrode pair (4') and a plurality of secondary electrodes (7) are connected to the electronic circuit (5) and disposed on a flat dielectric substrate (8). In a preferred embodiment of the invention, the dielectric substrate (8) is preferably made of a flexible biocompatible material, such as silicone, or of a conformable thermoplastic, such as polyethylene terephthalate. With this flat dielectric substrate (8), the implant device (1) may be positioned within the subject's body on natural layers, such as muscle fascia, or organ' surfaces through minimally invasive techniques.

Figure 5 shows a schematic representation of a lateral view of an embodiment of the invention, in which the implant device (1) has a substantially elongated flat shape and is implanted within a subject's body. In this embodiment, the implant device (1) is positioned on a fascia (i.e., sheath of stringy connective tissue that surrounds every part of a body), which is close to the treatment target area (2), which in this embodiment, comprises the target tissue (3). In another embodiment, the implant device (1) is positioned in an interface between two tissues of the subject's body.

The implant device (1) as illustrated in Figure 4, and Figure 5 comprises a substantially elongated flat shape. In another preferred embodiment of the invention, the implant device (1) may comprise a substantially elongated tubular shape.

The implant device (1) as illustrated in Figure 1, Figure 2, Figure 3, Figure 4, and Figure 5 is semi-flexible for minimal invasiveness. Said semi-flexibility is obtained by embedding the rigid elements (e.g., the components of the electronic circuit (5)) on or within a body made of a flexible material such as silicone.

Figure 6 shows the main elements comprised in a system (9) for delivering pulsed radiofrequency fields to a treatment target area (2) of a subject's body according to an embodiment of the invention, along with a representation of a preferred application of said system (9) for treating a condition. The system (9) comprises an implant device (1) according to any of the embodiments disclosed in this document, and an external unit (10). The external unit (10) comprises a plurality of external electrodes (11) and an external electronic circuit (12). The plurality of external electrodes (11) is adapted to encompass a region (13) of a subject's body comprising the treatment target area (2), wherein said treatment target area (2) comprises the target tissue (3). The external electronic circuit (12) comprises at least a generator (14), which is electrically connected to the plurality of external electrodes (11) and adapted to deliver electrical energy in the form of a voltage-controlled or a current-controlled waveform. This electrical energy is delivered in the form of radiofrequency pulses (16), i.e., it generates an alternating current (AC) electric field in the form of bursts. As seen in Figure 6, this is to be understood as meaning that the electric field (represented in Figure 6 by electric field lines (15)) generated between the plurality of external electrodes (11) is located in a region (13) of the subject's body in which the treatment target area (2) comprising the target tissue (3) is comprised. Thus, as the electrical energy passes through the tissues of the subject's body, the plurality of primary electrodes (4), specifically the volume conduction electrode pair (4'), pick-up and channel the plurality of radiofrequency electric current pulses (16) flowing through said tissue. In the present invention, this plurality of radiofrequency electric current pulses (16) picked-up by the volume conduction electrode pair (4') is designated as input. Once said input is conducted, it is regulated by the electronic circuit (5), delivering a pulsed radiofrequency current or voltage output, which is delivered to the treatment target area (2) of the subject's body through the treatment electrode (4"). Preferably, the radiofrequency electric current-controlled or voltage-controlled pulses (16) generated by the generator (14) have a fundamental frequency substantially between 50 kHz and 500 MHz and an electric field amplitude substantially between 1 V/cm and 1000 V/cm.

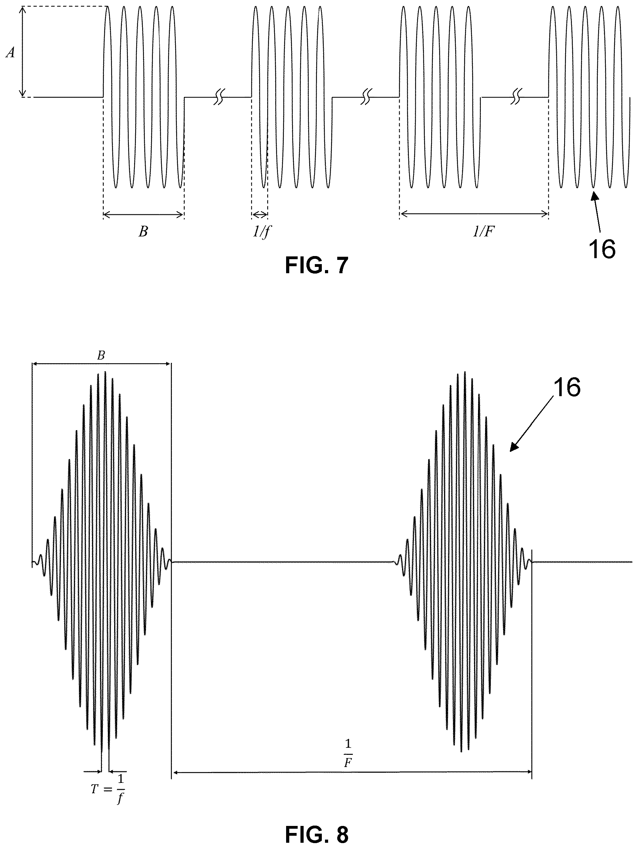

Figure 6 also presents a schematic view of the voltage-controlled or the current-controlled waveform that is supplied by the generator (14) to the plurality of external electrodes (11). This waveform is pulsed AC, i.e., an alternating current or voltage applied in the form of radiofrequency bursts (16). The main features of a pulsed radiofrequency waveform are shown in more detail in Figure 7. The fundamental frequency of the AC waveform is $f = \frac{1}{T}$, with T being the period of the oscillating waveform.

The fundamental frequency of the externally applied waveform is preferably comprised between substantially 50 kHz and 500 MHz. On the other hand, the resulting voltage amplitude (i.e., peak amplitude) of the AC waveform is comprised between substantially 20 V and 1000 V. This voltage range is apt for generating electric fields within tissues in the range from 1 V/cm to 1000 V/cm for external electrodes separation distances ranging from 1 cm to 100 cm. In turn, the 1 V/cm to 1000 V/cm electric field range is adequate for enabling the implant device (1), with separation distance between the centers of the volume conduction electrode pair (4') is between 1 cm and 50, and according to the embodiments described here, to generate output waveforms with an amplitude substantially between 5 V and 500 V.

Regarding the burst (16), they are characterized by two fundamental parameters: duration (B), and frequency of repetition ($F$). The product $F \times B$ corresponds to the so-called duty cycle (D).

The burst duration (B) and frequency of repetition (F) of the externally applied waveform are related to the burst duration (B) and repetition frequency (F) of the delivered electric fields to the treatment target area (2) according to regulation modes described below. Consequently, the treatment modality to be applied will determine these parameters.

The duty cycle of the pulsed AC electric fields is adjusted to prevent excessive heating of living tissues by resistive heating, but it can also be determined by the treatment modality.

In terms of adaptability, the generator (14) may comprise, according to an embodiment of the invention, modulating or windowing means to regulate the amplitude of the AC waveform within each pulse. These modulating means may be any standard modulating means known in the art. The modulation of the amplitude of the fields allows for a soft start and/or end of each pulse as seen in Figure 8. In said Figure, the effect of a Tukey window/modulation is represented, also known as the cosine-tapered window. Other known spectrally advantageous windows that may be applied in the present invention include the Hann window, the Hamming window, the Blackman window, the triangular window, the Gaussian window, and the Kaiser window.

Figures 6, 7, and 8 show pulsed sinusoidal waveforms. Nevertheless, other possible waveforms, such as biphasic rectangular or triangular waveforms, as long as they comprise no DC component, may also be used without departing from the scope of the invention.

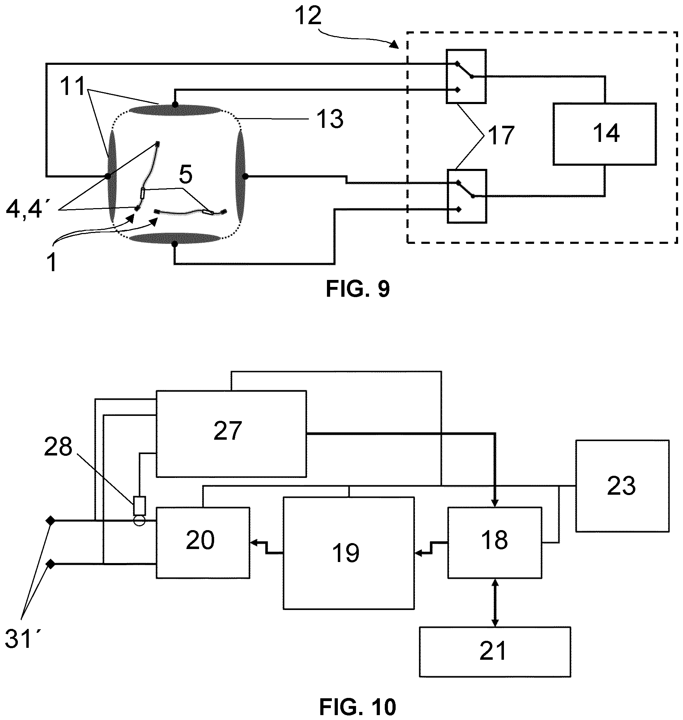

Figure 9 shows an embodiment of the system (9) of the invention in which the plurality of external electrodes (11) is arranged in pairs and are selected by means of switching elements (17) inserted in the connection between the generator (14) and said pair of electrodes (11). The switching elements (17) may consist in electromechanical relays or solid-state devices such as transistors and may be governed by a Digital Control Unit (DCU) to sequentially connect the output of the generator (14) to the pairs of external electrodes (11) (or to or to pairs of sets of external electrodes (11) in which each set is formed by short-circuiting the external electrodes (11) therein). Switching is produced in between bursts (16) and preferably, but not necessarily, after each burst (16). The amplitude of the current and/or voltage applied in each electrode combination can be adjusted for producing the same electric field.

Specifically, Figure 9 shows two pairs of external electrodes (11) arranged perpendicular to each other and the electric fields generated across each pair of external electrodes (11) overlap in a central region, wherein two implant devices (1) are located. In this embodiment, the AC electric fields are delivered through said pairs of external electrodes (11) and are switched to align the generated electric fields with the implant devices (1).

Regarding the generator (14), it may comprise several possible architectures within the scope of the present invention. It is known that electric fields are generated in living tissues when either a voltage-controlled waveform or a current-controlled waveform is delivered across electrodes.

In a preferred embodiment of the invention, the generator (14) comprises an external digital control unit (DCU) (18) such as a computer processing unit (CPU), a field-programmable gate array (FPGA) or any equivalent general digital microprocessor equipped with processing and timing means, adapted to autonomously govern the operation of a waveform generator (19) whose output is amplified by a voltage amplifier (20). The DCU (18) may also be connected to external communication means (21) in order to allow the exchange of information with the implant device (1), which according to an embodiment of the invention also comprises communication means (22). It may also be adapted to exchange information with a user of the system and/or to allow for manual control of the system by a user. It may also be adapted to be programmed with different treatment patterns (fundamental frequencies, amplitudes, duty cycles and duration of the active and rest phases) so that the whole treatment is delivered autonomously (i.e., without the intervention of a user). All the external electronic circuit (12) is powered by an external power stage (23).

The treatment can be initiated either manually by the user or automatically by the external unit (10), depending on the desired approach. When applied manually, the user simply attaches the external unit (10) and powers it on to activate the delivery of the voltage-controlled or current-controlled waveform. Alternatively, the treatment can be administered automatically at preprogrammed intervals, without user intervention, via the external unit (10). A variety of techniques for implementing timed actions are known in the art. These may include the use of hardware timers, software-based clock routines, or more sophisticated scheduling algorithms to ensure reliable and consistent delivery of the treatment at the desired intervals.

As mentioned above, the external unit (10) according to an embodiment of the invention is adapted to communicate with at least one implant device (1). The communication between the generator (14) and the implant device (1) may be done via volume conduction. In a further embodiment of the invention, the communication may be performed via other known methods for communication with implant devices such as far-field RF transmission with antennas, near-field transmission with coils, or optical transmission with optoelectronic elements. In contrast to communications by volume conduction, said methods (i.e., antennas, near-field transmission with coils or optical transmission with optoelectronic elements) can result in a bulkier implant device (1) as they imply embedding additional parts in the electronic circuit (5) of the implant device (1), like coils. However, these methods may provide advantages such as transmission at longer distances or higher transmission speeds.

According to an embodiment of the invention, the digital control unit (24) of the implant device (1) may comprise a computer processing unit (CPU), a field-programmable gate array (FPGA) or a digital microprocessor equipped with processing means to record and process electrical signals. The DCU (24) manages actions typical of a signal acquisition system and/or of a stimulation system. The DCU either executes an algorithm or interprets data and commands sent by the external unit (10) and, in response to those, executed preprogramed routines. The routines may comprise recording the temperature for a limited period, processing such recording (for instance, by obtaining the average temperature), and sending the result back to the external unit (10).

Volume conduction is suitable for powering the implant device (1) and for enabling bidirectional digital communications between the implant device (1) and the external unit (10). According to an embodiment of the invention, there are different communication methods, which can be either unidirectional (only from the external unit (10) to the implant device (1), or only from the implant device (1) to the external unit (10), or bidirectional (enabling an exchange of information between the external unit (10) and the implant device (1). Different methods are possible for implementing the so-called physical layer for both types of communication: downlink communication and/or uplink communication.

Downlink communication from the external unit (10) to the implant device (1): the high-frequency field generated by generator (14) comprised in the external electronic circuit (12) can be modulated using known methods (e.g., amplitude-shift key (ASK), frequency-shift key (FSK) and phaseshift key (PSK)) to transmit data from the external unit (10) to the implant device (1). After the modulation, the high-frequency current becomes a modulated management signal. By picking up the modulated signal across the volume conduction electrode pair (4'), the implant device (1) can recover the transmitted data by means of a demodulator (25). Since the main purpose of the high-frequency field generated by the external unit (10) is to transfer power to the implant device (1), it may be convenient to employ methods that minimize impact on power transfer. For instance, if ASK is employed, then Manchester codification is convenient as it provides self-clocking capabilities which are beneficial for demodulation and a constant average amplitude of the signal, which ensures power is being transferred. Alternatively, downlink communications can be performed in bursts different from those power transfer or can be performed only during a fraction of the power transfer burst. During said communications bursts or fractions of power transfer burst, the powering of the electronic circuit (5) of the implant device (1) can be sustained by means of a capacitive element comprised in the power stage (26), as it is commonly performed in voltage regulators.

Uplink communication from the implant device (1) to the external unit (10): two general methods are envisioned to perform data transmission from the implant device (1) to the external unit (10) (i.e, direct generation or load modulation). The direct generation is based on using known signal generation and modulation methods. In this way, the implant device (1) generates a voltage or current signal which is applied through the volume conduction electrode pair (4') thus generating an additional electric field that is transmitted by volume conduction through the surrounding of the treatment target area (2) (i.e., tissues). This additional electric field is then picked up by the plurality of external electrodes (11), which recovers the transmitted data, for example by means of a demodulator (25). On the other hand, in load modulation, the implant device (1) modulates the electrical load it exhibits for the high-frequency currents thus producing a communications signal. This can be accomplished, for instance, by means of switches (e.g. transistors) that partially short-circuit two electrodes of the implant device (1) thus creating a high-frequency electric field around the implant device (1) which is amplitude modulated. The external unit (10) can then detect the signal by means of known current sensing methods. For instance, by picking up the voltage across a resistor connected in series to the electrodes that deliver the high-frequency current.

Optionally the external electronic circuit (12) further comprises a monitoring unit (27) able to monitor one or more parameters in order to control the operation of the system (9). Said monitoring unit (27) is preferably connected to the output of the generator (14) and may include an electronic sensor (28) to monitor, for instance, the delivered current. Some examples of said parameters can be the temperature of the external electrodes (11), measured with temperature sensors on their surface or the total energy applied (i.e., product of time, voltage, current). The presented features regarding possible architectures of the external electronic circuit (12) (except for the switching elements (17) are schematically depicted in Figure 10.

All the features and embodiments detailed in the above paragraphs may be implemented in different configurations. More specifically, the implant device (1) and the system (9) can be arranged in various positions with respect to each other and with respect to subject's treatment target area (2), precisely depending on the specific application of the system (9).

In a further preferred embodiment of the invention, in order to avoid damage, the system (9) is adapted to control the applied current by the implant device (1). Two categories of treatment control are envisioned: control by the implant device (1) and control by the generator (14).

Within the control performed by the implant device (1), the implant device (1) controls the treatment by monitoring one or more parameters related to the treatment and by modulating, in response, the flow of current. Among these parameters can be the temperature sensed with an embedded temperature sensor (6) (e.g., a thermistor, a thermocouple, or a resistance temperature detector) or the applied energy in a time interval, which can be obtained by measuring the applied current (e.g. by measuring the voltage across a sensing resistor) and applied voltage. The modulation of the current flow may involve simply blocking the flow of current when a critical value (e.g., a temperature of 42 °C) is reached or modulating the resistance to current passage according to the output of PID controller implemented on a DCU. Other parameters that may be monitored and compared to a threshold or used as inputs of a more complex controller are the total treatment time, the time derivative of the temperature, the applied electric charge, and the electrical impedance of the tissues.

In contrast, within the control performed by the external generator (14), the generator (14) controls the treatment by monitoring one or more parameters related to the treatment and by modulating, in response, the delivery of the voltage or current bursts. Among these parameters can be the temperature sensed by the implant device (1) and communicated to the external generator (14). The modulation of the delivery of the voltage or current burst may involve simply stopping the delivery when a critical value (e.g., a temperature of 42 °C) is reached or modulating the amplitude or duration of the bursts according to the output of PID controller implemented on a DCU. Other parameters that may be monitored and compared to a threshold or used as inputs of a more complex controller are: the total treatment time, the total energy applied by the external generator (14), the time derivative of the temperature sensed by the implant device (1), the applied electric charge, the electrical impedance of the tissues sensed by the implant device (1), and the impedance across the plurality of external electrodes (11).

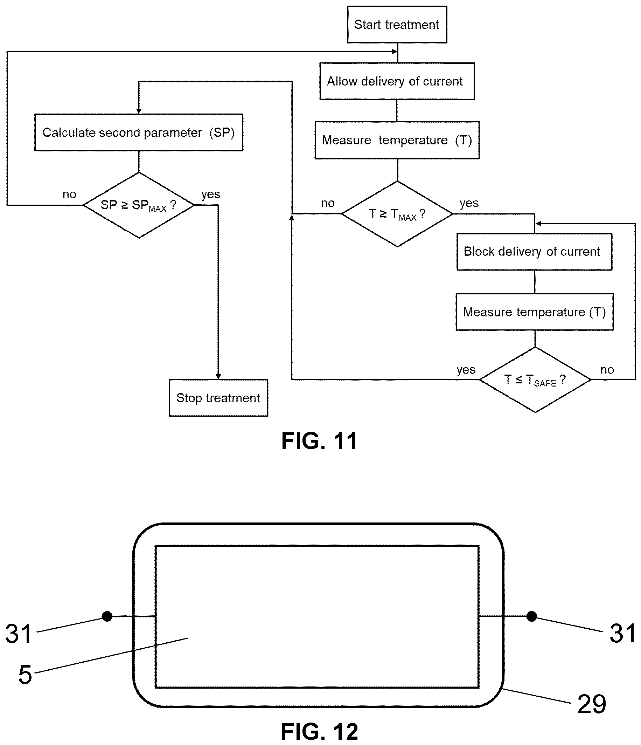

Figure 11 shows a process flow diagram to deliver pulsed radiofrequency fields in a controlled manner primarily intended to prevent damage in the treatment target area (2) and in its surroundings (i.e., tissues) in which the pulsed radiofrequency field is delivered according to an embodiment of the invention. This process is controlled by a digital control unit. Specifically, the process may be controlled by the digital control unit (24) of the implant device (1) or by the external digital control unit (18) of the external unit (10). As illustrated, when the treatment starts, the electronic circuit (5) of the implant device (1) conducts and regulates the input electrical energy, producing an output electrical energy, which is delivered to the treatment target area (2) of the subject's body through the treatment electrode (4"). The temperature (T) reached at the treatment target area (2) is measured by means of a temperature sensor (6) comprised in the implant device (1) and compared to a maximum temperature ($T_{max}$), being this $T_{max}$, established as the maximum temperature that can be reached at the treatment target area (2) during the treatment.

1) If $T \geq T_{max}$, the digital control unit (18, 24) stops the delivery of the pulsed radiofrequency field, either from the external unit (10) or from the implant device (1), and then the temperature (T) is measured again. Said temperature is compared to a reference safe temperature ($T_{safe}$) in order to determine whether the treatment can resume.

    a. If $T \leq T_{safe}$, a second parameter (SP) is calculated, then being compared to a maximum second parameter threshold ($SP_{max}$).

        i. If $SP \geq SP_{max}$, the digital control unit (18, 24) stops the delivery of the pulsed radiofrequency field;
        ii. If $SP \leq SP_{max}$, the delivery of the pulsed radiofrequency field continues, and the control process starts again as being described above, by measuring T at the treatment target area (2), by the temperature sensor (6) of the implant device (1) comprised in the system (9);

    b. If $T \geq T_{safe}$, the digital control unit (18, 24) stops the delivery of pulsed radiofrequency field and then step 1) is repeated.

2) If $T \leq T_{max}$, a second parameter (SP) is calculated, then being compared to a maximum second parameter threshold (SP).

        i. If $SP \geq SP_{max}$, the digital control unit (18, 24) stops the delivery of the pulsed radiofrequency field; and
        ii. If $SP \leq SP_{max}$, the delivery of the pulsed radiofrequency field continues, and the control process starts again as being described above, by measuring T at the treatment target area (2), by the temperature sensor (6) of the implant device (1) comprised in the system (9).

In a further embodiment of the invention, the second parameter (S) may comprise the applied energy in a time interval, either by the external unit (10) or by the implant device (1), which can be obtained by measuring the applied current (e.g. by measuring the voltage across a sensing resistor) and the applied voltage, or may comprise other parameters such as the total treatment time, the time derivative of the temperature, the applied electric charge, and the electrical impedance of the tissues.

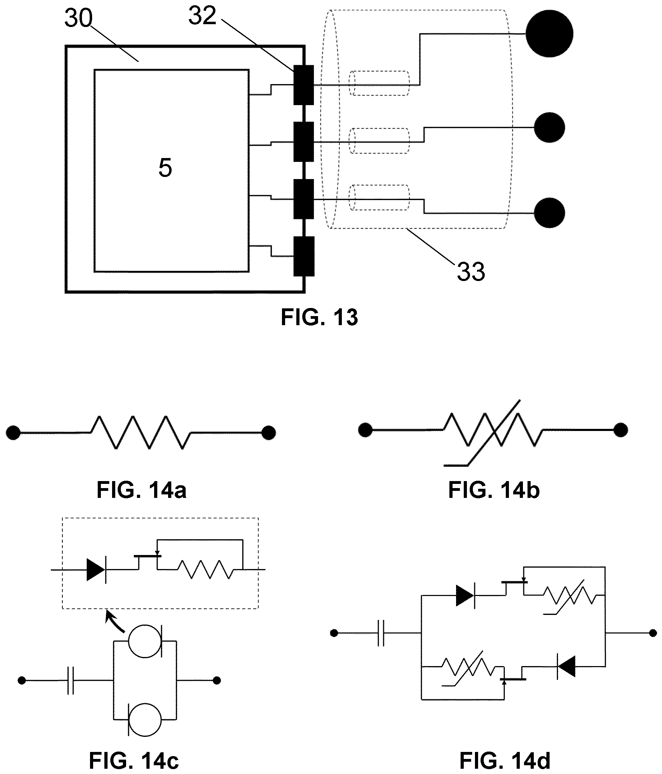

Figure 12 illustrates the electronic circuit (5) of the implant device (1) according to an embodiment of the invention, in which the electronic circuit (5) is enclosed into an electronic package (29), preferably made by a hermetic capsule (30), and has at least two circuit terminals (31) adapted to connect the electronic circuit (5) to the plurality of primary electrodes (4). In a preferred embodiment of the invention (see Figure 13), the hermetic capsule (30) is electrically connected by means of hermetic electrical connections (32) and through feedthroughs to wires comprised in an insulation capsule (i.e., insulation of the electrical connections) (33) connected to the plurality of primary electrodes (4) and to the plurality of secondary electrodes (7).

As mentioned above, the implant device (1) delivers the pulsed radiofrequency electric fields (i.e., output electrical energy) to the treatment target area (2) of the subject's body. In a preferred embodiment of the invention, to obtain the output, the AC fields generated by the generator (14) (i.e., the input) of the system (9) may be regulated through the implant device (1). For this purpose, two regulation modes can be followed, which are referred to herein as direct mode or conversion mode.

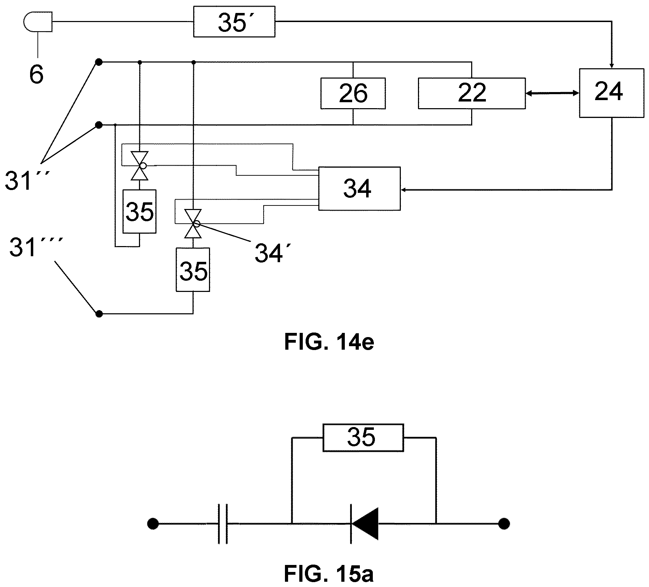

In direct mode, the fundamental frequency of the externally applied voltages or currents by the generator (14) is of intermediate frequency (i.e., between 50 kHz and 1 MHz) and the implant device (1) acts as a conduit adapted to limit the current that flows through its plurality of electrodes. For this, according to an embodiment of the invention (see Figure 14a), the electronic circuit (5) of the implant device (1) comprises a current limiting circuit (CLC) (35) to limit in a controlled way the amount of alternating current that flows through them, independently of the load and the applied voltage. In a preferred embodiment of the invention, the CLC (35) can comprise different topologies (see Figure 14 a-d), such as a positive temperature coefficient (PCT) resistor, two antiparallel current limiting diodes (CLD), two antiparallel CLDs with PCT resistors, or a digitally controllable current-limiting circuit. A PCT resistor, such as a PTC thermistor, limits the current flowing through it by increasing its resistance as the current increases. This is because higher current leads to more heating, which in turn raises the temperature, thus increasing resistor's resistance. In a preferred embodiment, the PTC resistor may be positioned close to the treatment electrode (4") to limit current through both the self-heating of the CLD and the rise in tissue temperature. Thus, this topology may function both as a CLC (35) and as a treatment control mechanism. Two antiparallel current limiting diodes (CLD) may be implemented using a diode, a transistor, and a resistor, which limit the alternating currents by each CLD being activated during a half-cycle of the waveform. Preferably, a capacitor is mounted in series with the two antiparallel CLDs to block DC currents that may be originated due to unmatching between the two CLDs. Another topology may be two antiparallel CLDs with PTC resistors, acting as temperature sensors that set the current limit. The resistance in CLDs implemented using a diode, a transistor, and a resistor sets the current limit of the CLDs. If, instead of regular resistors, PTC resistors are used, the current limit will depend on the temperature. By placing the PTC resistors close to the treatment electrode (4"), the temperature of the treatment is controlled, since as tissue temperature rises, the current limit is reduced, thus limiting the flow of current and the associated tissue heating. As for the digitally controllable current-limiting circuit (see Figure 14 e), it is possible to implement a digital circuit, for instance, a state-machine implemented in an integrated circuit, which is powered by extracting part of the current flowing through the implant device (1) and which is able to activate gates (34) that control the flow of current following preestablished patterns, in response to events (e.g. reaching a temperature) or to commands sent externally, which are manipulated by a signal conditioning circuit (35').

In conversion mode, the externally applied voltages or currents are of high-frequency (i.e., the fundamental frequency is higher than 1 MHz) to prevent unsought stimulation of excitable tissues outside treatment target area (2) and are delivered as trains of burst. In this mode, the implant device (1) acts as a conduit that converts the applied high-frequency currents into intermediate frequencies for an effective treatment. For this, in a preferred embodiment, the electronic circuit (5) comprises a conversion circuit. This mode can be distinguished in conversion rectifier mode or in conversion pulsing mode. Thus, in a preferred embodiment of the invention, depending on the mode, the conversion circuit comprises a rectifier (in those cases in which the mode is based on a conversion rectifier) or a pulse generator (36) (in those cases in which the mode is based on conversion pulsing).

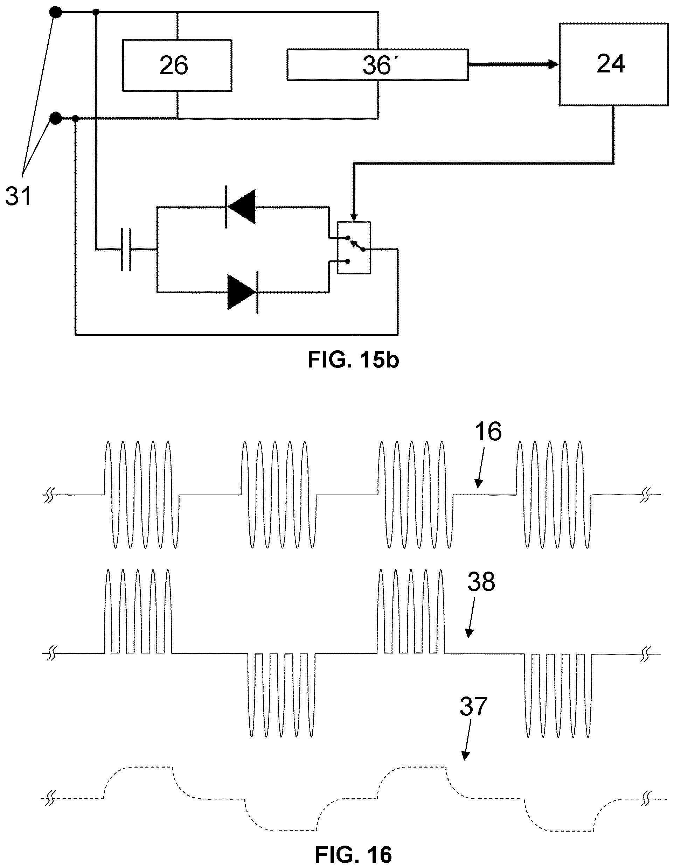

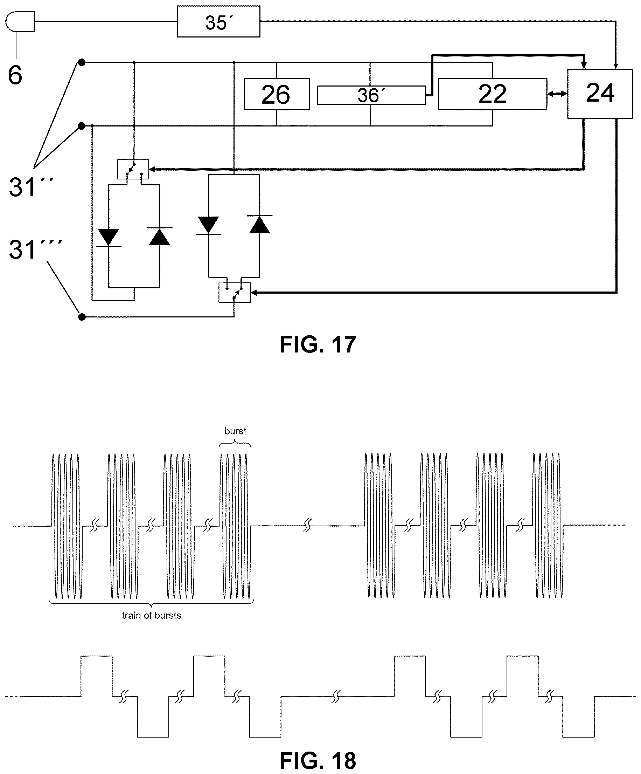

In the conversion rectifier mode, the implant device (1) rectifies the high-frequency currents that picks-up with its volume conduction electrode pair (4'). This produces currents existing the implant device (1) with spectral contents at intermediate frequencies so that treatment can be achieved. To perform the rectification, the electronic circuit (5) of the implant device (1) according to an embodiment of the invention, comprises a single diode (preferably with a capacitor mounted in series with the diode to prevent dc currents and a resistor in parallel to allow capacitor discharge, see Figure 15a). In another embodiment, the electronic circuit (5) is adapted to operate as a diode that toggles its polarity after each burst (see Figure 15b), wherein each burst is measured by a burst detector (36'). In a further embodiment, diode toggling is performed in digitally controllable smart rectifier that, in addition, may include other functionalities such as communications and sensing (see Figure 17). Figure 16 shows how the conversion rectifier mode operates when the electronic circuit (5) of the implant device (1) acts as a diode that toggles its polarity after each burst. The bottom trace represents a low-pass filtered (LPF) (37) version of the intermediate trace (38), which is the current generated by the implant device (1). The LPF version is relevant because the cell membrane acts as a low-pass filter. Thus, this trace corresponds to the waveform that would be perceived by the cells.

In the conversion pulsing mode, the implant device (1), through its volume conduction electrode pair (4'), draws power from the high-frequency currents delivered by the generator (14) to charge a capacitor whose energy is used to deliver intermediate frequency currents so that treatment can be achieved. In a preferred embodiment, the intermediate frequency currents consist of individual pulses (16) which are generated after each burst. This allows for a relatively small capacitor because only the charge required to deliver a pulse is necessary, rather than the charge necessary for delivering an entire burst of pulses.

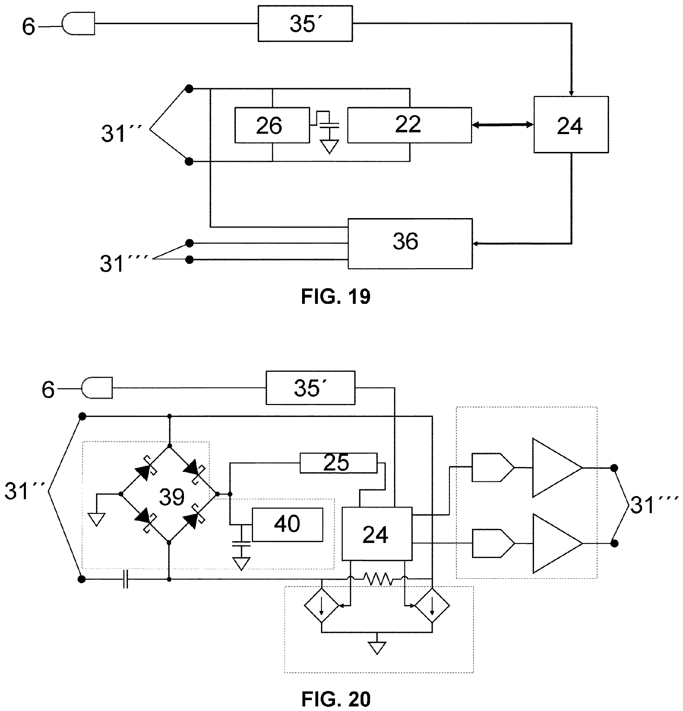

Figure 18 illustrates how the conversion pulsing mode operates in said preferred embodiment. As it can be appreciated, the waveform applied by the generator (14) comprised in the external unit (10) of the system (9), consists of trains of burst (not of continuous burst) and each burst is transformed into a semicycle of the alternating waveform produced by the implant device (1). The generated pulses can be square monophasic, but they may also take other forms, like square monophasic pulses of alternating phase (i.e., positive and negative), biphasic pulses, triangular pulses, and sinusoidal pulses. These pulses can be voltage or current controlled. Figure 19 illustrates a possible topology for the electronic circuit (5) of the implant device (1) according to an embodiment of the invention for conversion pulsing mode. In this embodiment, the elements of the electronic circuit (5) are powered by the power extracted by the power stage (26), which stores energy in an energy storage capacitor to be used after each burst. The power stage (26) extracts power by collecting at least part of the input, which is generated by the generator (14) of the external unit (10), which flows through the tissues of the subject's body by volume conduction. In this embodiment, the electronic circuit (5) comprises at least two circuit terminals (31"), which ones are used for connecting the electronic circuit (5) to the plurality of primary electrodes (4). Furthermore, in a preferred embodiment of the invention, wherein the implant device (1) comprises at least a plurality of secondary electrodes (7), the electronic circuit (5) also has at least two circuit terminals (31‴) for connecting the electronic circuit (5) to said plurality of secondary electrodes (7).

Figure 20 illustrates another topology for the electronic circuit (5) of the implant device (1) according to an embodiment of the invention for conversion pulsing mode. In this embodiment, the electronic circuit (5) comprises at least two circuit terminals (31"), which are used for connecting the electronic circuit (5) to the plurality of primary electrodes (4). Furthermore, in a preferred embodiment of the invention, wherein the implant device (1) comprises at least a plurality of secondary electrodes (7), the electronic circuit (5) also has at least an additional circuit terminal (31‴) for connecting the electronic circuit (5) to said plurality of secondary electrodes (7). The plurality of primary electrodes (4), which preferably correspond to the most separated electrodes of the implant device (1), pick up a part of the high-frequency current flowing across the tissues of a subject's body. By means of a full bridge rectifier (39) and a voltage regulator (40), this high-frequency current is transformed into dc voltage to power all the elements of the electronic circuit (5). A blocking capacitor is connected in series with the plurality of primary electrodes (4), adapted to prevent and to enable the flow of high-frequency current to and from the electronic circuit (5). The blocking capacitor is typically required to prevent the passage of very low frequency currents, essentially dec, that would cause electrochemical reactions at the plurality of primary electrodes (4) and could damage both the surrounding living tissue and the electrodes themselves. A demodulator (25) performs demodulation of the signal embedded with the ac waveform that is used to power the implant device (1). This signal can be, for instance, modulated in amplitude and it can contain digital data such as the address of the implant device (1) that external unit (10) wants to communicate, commands for stimulation, or instructions for controlling the communications flow between the implant device (1) and the external unit (10).

A system (9) according to any of the above-described embodiments may be operated as one or more steps of a method for treating a treatment target area (2) of a subject's body based on the delivery of radiofrequency electric current pulses (16) to a treatment target area (2) of the subject's body.

This method comprises deploying the implant device (1) in a treatment target area (2) of a subject's body, deploying the plurality of external electrodes (11) in a region (13) of the subject's body, connecting the plurality of external electrodes (11) to the external electronic circuit (12) and continuously supplying radiofrequency electric current pulses (16) from the generator to the plurality of external electrodes (11), wherein said radiofrequency electric current pulses (16) comprise the above-mentioned characteristics: a fundamental frequency between 50 kHz and 500 MHz and an amplitude between 20 V and 1000 V.

In a further preferred embodiment of the invention, within the disclosed method, the method further comprises a stimulation step, which is performed prior to the delivery of the pulsed radiofrequency fields, in order to confirm the proper deployment of the implant device (1). With it is possible to ensure that the implant device (1) is correctly aligned with the target area, thereby maximizing the effectiveness of the treatment. The stimulation step comprises the delivery of lower frequency currents by the implant device. This may be performed by allowing the implant device (1) to deliver currents of lower frequency. In the direct regulation mode this can be achieved by lowering the fundamental frequency of the externally applied voltages or currents to the range from 1 Hz to 1 kHz and, preferably, in the range from 10 Hz to 100 Hz. These low-frequency voltages or currents can be delivered either continuously or in the form of bursts. Similarly, in the conversion regulation mode, stimulation can be achieved by lowering the repetition frequency of the bursts to the range from 1 Hz to 1 kHz and, preferably, in the range from 10 Hz to 100 Hz. These can be delivered either continuously or in the form of trains of bursts.

In a further preferred embodiment of the invention, within the disclosed method, the target tissue (3) comprises one or more target nerves. Thus, with the proposed method it is possible to treat a disorder caused by the target nerve or nerves.

**Claims**

1.  An implant device (1), adapted to be implanted inside a subject's body for delivering pulsed radiofrequency electric fields to a treatment target area (2) of said subject's body, comprising at least:

    - a plurality of primary electrodes (4), comprising at least a volume conduction electrode pair (4') adapted to pick-up and channel an input electrical energy flowing through a tissue of the subject's body by volume conduction in the form of a plurality of radiofrequency electric current pulses (16), wherein the at least volume conduction electrode pair (4') comprises a treatment electrode (4") and a return electrode (4'''); and
    - an electronic circuit (5) electrically connected to the plurality of primary electrodes (4) and adapted to conduct and regulate the input electrical energy, and deliver an output electrical energy in the form of a plurality of radiofrequency pulses;

    wherein the electrical energy flowing through the tissue of the subject's body in the form of a plurality of radiofrequency electric current pulses (16) comprises a fundamental frequency substantially between 50 kHz and 500 MHz and an electric field amplitude substantially between 1 V/cm and 1000 V/cm; and **characterized in that** the plurality of primary electrodes (4) and the electronic circuit (5) are further adapted to deliver pulsed radiofrequency electric fields to the treatment target area (2) of the subject's body through the treatment electrode (4"), and wherein a resulting voltage waveform between the treatment electrode (4") and the return electrode (4''') comprises an amplitude substantially between 5 V and 500 V.

2.  An implant device (1) according to the preceding claim, wherein the electronic circuit (5) further comprises the following elements:

    - a digital control unit (24) adapted to control the delivery of the pulsed radiofrequency electric fields to the treatment target area (2); and
    - a power stage (26) adapted to extract a power by collecting at least part of the input electrical energy and to supply this power to the elements comprised within the electronic circuit (5).

3.  An implant device (1) according to the preceding claim, wherein the electronic circuit (5) further comprises communications means (22) adapted to receive or send information.

4.  An implant device (1) according to any one of the claims 1 to 3, further comprising monitoring means adapted to monitor one or more parameters related to the delivery of pulsed radiofrequency electric fields to the treatment target area (2) and/or to the tissue of the subject's body.

5.  An implant device (1) according to the preceding claim, wherein the monitoring means comprises a temperature sensor (6).

6.  An implant device (1) according to any one of claims 1 to 5, wherein the implant device (1) comprises an elongated flat shape or an elongated tubular shape.

7.  An implant device (1) according to any one of claims 1 to 6, wherein the electronic circuit (5) and the electrodes (4) are embedded into a flexible material.

8.  An implant device (1) according to any one of claims 1 to 7, wherein the electronic circuit (5) comprises a current limiting circuit (35) adapted to limit the flow of the radiofrequency electric current pulses (16).

9.  An implant device (1) according to the preceding claim, wherein the current limiting circuit (35) comprises a positive temperature coefficient (PCT) resistor, two antiparallel current limiting diodes (CLD), two antiparallel CLDs with PCT resistors, or a digitally controllable current-limiting circuit.

10. An implant device (1) according to any one of claims 1 to 9, wherein the electronic circuit (5) comprises a conversion circuit.

11. An implant device (1) according to the preceding claim, wherein the conversion circuit comprises a rectifier or a pulse generator (36).

12. A system for delivering pulsed radiofrequency fields to a treatment target area (2) of a subject's body, comprising at least an implant device (1) according to any one of claims 1 to 11 and an external unit (10), wherein said external unit (10) comprises:

   - a plurality of external electrodes (11) adapted to encompass a region (13) of a subject's body comprising a treatment target area (2); and
   - an external electronic circuit (12) comprising at least a generator (14) electrically connected to the plurality of external electrodes (11);

   and **characterized in that** the generator (14) is adapted to generate a plurality of radiofrequency electric pulses (16) comprising:

   - a fundamental frequency substantially between 50 kHz and 500 MHz; and/or
   - a resulting voltage amplitude substantially between 20 V and 1000 V.

13. A system (9) according to the preceding claim, wherein the external electronic circuit (12) comprises at least the following elements:

   - an external digital control unit (18) adapted to automatically control the delivery of the plurality of radiofrequency electric pulses (16) generated by the generator (14); and
   - an external power stage (23) adapted to power the elements comprised within the external electronic circuit (12).

14. A system (9) according to the preceding claim, wherein the external electronic circuit (12) comprises communications means (22) adapted to receive information generated by the implant device (1) or adapted to send information to the implant device (1).

15. A method for controlling the delivery of pulsed radiofrequency electric fields delivered by an implant device (1) in a system (9) according to any one of claims 12 to 14, wherein the method comprises the realization of the following steps:

   - establishing a maximum temperature ($T_{max}$) that can be reached at a treatment target area (2), and a reference safe temperature ($T_{safe}$);
   - measuring a temperature (T) at the treatment target area (2), by means of a temperature sensor (6) comprised in the implant device (1);
   - transferring the information, related to the T measured, to a digital control unit (24) comprised in the implant device (1), or to an external digital control unit (18);
   - comparing T to $T_{max}$, by the digital control unit (18, 24);

   1) If $T \geq T_{max}$, the digital control unit (18, 24) stops the delivery of the pulsed radiofrequency field and measures again the T, and then compares said T to the $T_{safe}$;

   a) If $T \leq T_{safe}$, a second parameter (SP) is calculated, then being compared to a maximum second parameter threshold ($SP_{max}$);

   i. If $SP \geq SP_{max}$, the digital control unit (18, 24) stops the delivery of the pulsed radiofrequency field;
   ii. If $SP \leq SP_{max}$, the delivery of the pulsed radiofrequency field continues, and the control process starts again by measuring T at the treatment target area (2), by the temperature sensor (6) of the implant device (1) comprised in the system (9);

   b) If $T \geq T_{safe}$, the digital control unit (18, 24) stops the delivery of pulsed radiofrequency field and then step 1) is repeated.

   2) If $T \leq T_{max}$, a second parameter (SP) is calculated, then being compared to a maximum second parameter threshold (SP):

i. If $SP \geq SP_{max}$, the digital control unit (18, 24) stops the delivery of the pulsed radiofrequency field; and

ii. If $SP \leq SP_{max}$, the delivery of the pulsed radiofrequency field continues, and the control process starts again by measuring T at the treatment target area (2), by the temperature sensor (6) of the implant device (1) comprised in the system (9).

**FIG. 1**

**FIG. 2**

**FIG. 3**

8     4,4′     1

5

7

**FIG. 4**

7    5    1

8

3

4,4′

**FIG. 5**

10

12

11

16

9

3

4,4′

4″     5     4‴

15

1

**FIG. 6**

**FIG. 7**

**FIG. 8**

FIG. 9

FIG. 10

Start treatment

Allow delivery of current

Calculate second parameter (SP)

Measure temperature (T)

no ← SP ≥ SP$_{MAX}$ ? → yes

no ← T ≥ T$_{MAX}$ ? → yes

Block delivery of current

Measure temperature (T)

yes ← T ≤ T$_{SAFE}$ ? → no

Stop treatment

**FIG. 11**

31

5

31

29

**FIG. 12**

30  32

5

33

**FIG. 13**

**FIG. 14a**

**FIG. 14b**

**FIG. 14c**

**FIG. 14d**

**FIG. 14e**

**FIG. 15a**

**FIG. 15b**

**FIG. 16**

35´

6

26    36´    22    24

31´´

31´´´

**FIG. 17**

burst

train of bursts

**FIG. 18**

**FIG. 19**

**FIG. 20**

Europäisches
Patentamt

European
Patent Office

Office européen
des brevets

# EUROPEAN SEARCH REPORT

**Application Number**

EP 24 38 3161

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2016/346537 A1 (MASHIACH ADI [IL]) 1 December 2016 (2016-12-01) | 1-11 | INV. A61N1/36 |
| A | * the whole document * | 12-15 | A61N1/32 A61B18/12 |
| | ----- | | |
| A | US 2010/016929 A1 (PROCHAZKA ARTHUR [CA]) 21 January 2010 (2010-01-21) * the whole document * | 12-15 | |
| | ----- | | |
| A | US 2014/058481 A1 (PERRYMAN LAURA TYLER [US] ET AL) 27 February 2014 (2014-02-27) * paragraphs [0003] - [0011], [0109], [0110]; figures 1, 12, 13 * | 1-15 | |
| | ----- | | |

**TECHNICAL FIELDS SEARCHED (IPC)**

A61B
A61N

The present search report has been drawn up for all claims

| Place of search | Date of completion of the search | Examiner |
|---|---|---|
| Munich | 27 March 2025 | Lorenz, Larissa |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

..........................................................................................

& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 24 38 3161

This annex lists the patent family members relating to the patent documents cited in the above-mentioned European search report.
The members are as contained in the European Patent Office EDP file on
The European Patent Office is in no way liable for these particulars which are merely given for the purpose of information.

27-03-2025

| Patent document cited in search report | Publication date | Patent family member(s) | | Publication date |
|---|---|---|---|---|
| US 2016346537 A1 | 01-12-2016 | US | 2014039579 A1 | 06-02-2014 |
| | | US | 2014379049 A1 | 25-12-2014 |
| | | US | 2016346537 A1 | 01-12-2016 |
| | | US | 2018221657 A1 | 09-08-2018 |
| US 2010016929 A1 | 21-01-2010 | US | 2010016929 A1 | 21-01-2010 |
| | | WO | 2011035421 A1 | 31-03-2011 |
| US 2014058481 A1 | 27-02-2014 | AU | 2012211055 A1 | 27-06-2013 |
| | | BR | 112013018470 A2 | 18-10-2016 |
| | | CA | 2831062 A1 | 02-08-2012 |
| | | CN | 103796715 A | 14-05-2014 |
| | | CN | 106902457 A | 30-06-2017 |
| | | EP | 2667942 A2 | 04-12-2013 |
| | | EP | 3685880 A1 | 29-07-2020 |
| | | EP | 3821941 A1 | 19-05-2021 |
| | | EP | 4424360 A2 | 04-09-2024 |
| | | ES | 2988349 T3 | 20-11-2024 |
| | | JP | 6076915 B2 | 08-02-2017 |
| | | JP | 2014513562 A | 05-06-2014 |
| | | MX | 340144 B | 15-06-2016 |
| | | US | 2012283800 A1 | 08-11-2012 |
| | | US | 2014058481 A1 | 27-02-2014 |
| | | US | 2016367825 A1 | 22-12-2016 |
| | | US | 2018236248 A1 | 23-08-2018 |
| | | WO | 2012103519 A2 | 02-08-2012 |

EPO FORM P0459

For more details about this annex : see Official Journal of the European Patent Office, No. 12/82